# EUROPÄISCHE PATENTSCHRIFT

(11) **EP 4 072 635 B1**
(45) Veröffentlichungstag und Bekanntmachung des Hinweises auf die Patenterteilung: **24.09.2025**
(21) Anmeldenummer: 20820802.5
(22) Anmeldetag: 02.12.2020
(51) Int. Cl.: A61M 15/00, A61K 9/00

(54) **PULVERFÖRMIGE FORMULIERUNGEN ZUR INHALATION**
POWDER FORMULATIONS FOR INHALATION
FORMULATIONS DE POUDRE À INHALER

(30) Priorität: 10.12.2019 DE 102019219277
(43) Veröffentlichungstag der Anmeldung: 19.10.2022
(73) Patentinhaber: Christian-Albrechts-Universität zu Kiel, 24118 Kiel (DE)
(72) Erfinder: SCHERLIESS, Regina, 24118 Kiel (DE); BOCK, Simon Stefan, 24118 Kiel (DE)
(74) Vertreter: Taruttis, Stefan Georg
(86) Internationale Anmeldenummer: PCT/EP2020/084271
(87) Internationale Veröffentlichungsnummer: WO 2021/115877

(56) Entgegenhaltungen:
- EP-A1- 0 714 313
- EP-A2- 2 526 990
- US-A1- 2001 027 790

## Beschreibung

Die vorliegende Erfindung betrifft pulverförmige pharmazeutische Formulierungen, die einen partikelförmigen Wirkstoff und Trägerpartikel enthalten, an denen der Wirkstoff bevorzugt nur oberflächlich haftet, sowie ein Verfahren zur Herstellung solcher Formulierungen. Die Formulierungen zeichnen sich dadurch aus, dass sie ein Mischelement enthalten, das in der Formulierung frei beweglich ist und eine Dispergierhilfe für den partikelförmigen Wirkstoff und die Trägerpartikel bildet. Alternativ dazu, dass das Mischelement in der Formulierung enthalten ist, kann das Mischelement in einem Abschnitt des Strömungskanals eines Inhalators angeordnet sein, z.B. frei beweglich in einer Kammer, die von Trägergas und pulverförmiger pharmazeutischer Formulierung durchströmt wird. Die Erfindung betrifft auch das Mischelement sowie ein Verfahren zu dessen Herstellung und des Weiteren bevorzugte Trägerpartikel und Verfahren zu deren Herstellung.

Das Mischelement hat den Vorteil, bei Verwirbelung der Formulierung mittels eines Trägergases, z.B. bei der Erzeugung einer Partikelwolke bei der Inhalation, aus einer pulverförmigen Formulierung einen größeren Anteil an Feinpartikeln in dem Trägergas zu verteilen, die eine für das Eindringen in die Lunge geeignete Größe von z.B. maximal 5 µm oder darunter aufweisen.

### Stand der Technik

EP 2 526 990 A2, US 2001/027790 A1 und EP 0 714 313 A1 beschreiben zur Inhalation pulverförmiger pharmazeutischer Formulierungen kugelförmige Mischelemente.

Hickey, A., KONA Powder and Particle Journal 35 (2018) 3-13 beschreibt zur Inhalation pulverförmige pharmazeutische Formulierungen, die als Trägerpartikel Laktosekristalle, z.B. gemahlen und gesiebt, aufweisen und daran anhaftend separat sprühgetrockneten oder mikronisierten Wirkstoff.

Renner et al., International Journal of Pharmaceutics 20-29 (2017) beschreiben das aerodynamische Verhalten von interaktiven Pulvermischungen mit Glaskugeln als Trägerpartikeln, die als Modell für Trägerpartikel in pulverförmigen Formulierungen mit separat sprühgetrocknetem Wirkstoff dienten, der ohne weiteres durch Mischen oberflächlich an diesen Trägerpartikeln anhaftete.

### Aufgabe der Erfindung

Der Erfindung stellt sich die Aufgabe, eine alternative pulverförmige pharmazeutische Formulierung und ein Verfahren zu deren Herstellung bereitzustellen. Die Formulierung soll bevorzugt beim Verteilen einen möglichst hohen Anteil an partikelförmigem Wirkstoff einer aerodynamischen Partikelgröße von maximal 5 µm erzeugen, um die Wirkstoffpartikel inhalierbar bzw. lungengängig zu machen. Eine weitere Aufgabe liegt in der Bereitstellung eines Mischelements, das zur Fluidisierung von partikelförmigem Wirkstoff einer Partikelgröße von maximal 5 µm, insbesondere aus einer pulverförmigen pharmazeutischen Formulierung im Trägergasstrom geeignet ist, sowie in der Bereitstellung eines Herstellungsverfahrens für solche Mischelemente.

### Beschreibung der Erfindung

Die Erfindung löst die Aufgabe mit den Merkmalen der Ansprüche und insbesondere mit einer pulverförmigen pharmazeutischen Formulierung, die zusätzlich zu Trägerpartikeln und partikelförmigem Wirkstoff, der bevorzugt an der Oberfläche der Trägerpartikel anhaftet, ein Mischelement enthält, wobei die im Trägergasstrom fluidisierte Formulierung das Mischelement enthalten kann. Die Trägerpartikel weisen bevorzugt ein und dieselbe Form und dieselbe Größe auf, so dass die Trägerpartikel in Form und Größe einheitlich sind. Bevorzugt ist die pulverförmige pharmazeutische Formulierung als eine Einzeldosis konfektioniert, z.B. als Einzeldosis in einen Behälter abgefüllt, z.B. in einer Kapsel. Alternativ kann die Formulierung, die aus partikelförmigem Wirkstoff und Trägerpartikeln besteht, in einem Reservoir im Inhalator enthalten sein, aus dem vor dem Dispergieren bzw. Fluidisieren eine Einzeldosis abgeteilt wird.

Das Mischelement hat in einer ersten Dimension eine Größe von zumindest 1 mm, bevorzugt von zumindest 1,5 mm oder von zumindest 2 mm, z.B. bis 10 mm oder bis 9 mm oder bis 8 mm oder bis 7 mm, und in den beiden anderen Dimensionen eine Größe von zumindest 50%, bevorzugt zumindest 60%, zumindest 70%, zumindest 75%, zumindest 80% oder zumindest 90% der Größe, die es in der ersten Dimension aufweist.

Generell bevorzugt weist die Formulierung den Wirkstoff in Partikelgrößen der einzelnen Partikel von maximal 5 µm auf, wobei Partikel in der Formulierung agglomeriert sein können, z.B. an der Oberfläche von Trägerpartikeln anhaftend.

Bevorzugt weist das in der pulverförmigen pharmazeutischen Formulierung enthaltene Mischelement um einen Hohlraum angeordnete Wände auf, die zumindest eine zum Hohlraum offene Durchbrechung aufweisen. Die Wände umfassen die zumindest eine Durchbrechung und spannen zwischen sich einen Hohlraum auf, so dass der Hohlraum durch die zumindest eine Durchbrechung offen ist. Bevorzugt weist das Mischelement um den Hohlraum angeordnete Wände auf, die zumindest zwei an gegenüberliegenden Wänden angeordnete und zum Hohlraum offene Durchbrechungen aufweisen. Die Wände des Mischelements weisen zwischen ihrer Außenfläche und dem Hohlraum eine Dicke von z.B. maximal 20% oder von maximal 15% oder von maximal 10% der Größe des Mischelements in dieser Dimension auf. Die Wände können auf ihren dem Hohlraum gegenüberliegenden Flächen eine Gesamtfläche von maximal 50%, maximal 40%, maximal 30% oder maximal 20% oder maximal 10% der Gesamtfläche der zumindest einen Durchbrechung aufweisen, die von den Wänden aufgespannt wird. Dabei können die Wände zusammen eine gegenüber dem Hohlraum bestimmte Gesamtfläche einnehmen, die maximal 50% der von der zumindest einen Durchbrechung aufgespannten Fläche beträgt, so dass die Wände einen kleineren Anteil des Hohlraums überdecken, als die zumindest eine Ausnehmung den Hohlraum überspannt. Optional kann das Mischelement Wände aufweisen, die ein Abrollen, insbesondere ein kontinuierliches Abrollen, entlang Innenflächen eines Behälters erlauben, der eine Dispergierkammer oder Fluidisierungskammer sein kann, z.B. im Strömungsweg bzw. Strömungskanal eines Inhalators, oder ein Vorratsbehälter. Die Wände des Mischelements können z.B. auf ihren dem Hohlraum gegenüberliegende Flächen konvex gebogen sein. Dabei können die dem Hohlraum gegenüberliegenden Flächen der Wände, die die Außenflächen der Wände bzw. des Mischelements bilden, kontinuierlich oder abschnittsweise ausgebildet sein. Die kontinuierlich ausgebildeten, konvex gebogenen Flächen können sich z.B. über zumindest ¼, 1/3 oder ½ des Umfangs erstrecken.

Es hat sich gezeigt, dass diese Ausführungsform des Mischelements bei der Verwirbelung der pulverförmigen Zusammensetzung mit einem Trägergas eine höhere Fluidisierung von Partikeln bewirkt, die kleiner als 5 µm sind, z.B. im Vergleich mit der Fluidisierung einer ansonsten gleichen Formulierung ohne Mischelement. Die höhere Fluidisierung kleiner Partikel wird gegenwärtig auf die dispergierende Wirkung des Mischelements auf die Formulierung zurückgeführt.

Dabei kann das Mischelement Wände aufweisen, die von einem oder von zumindest zwei miteinander verbundenen, jeweils in sich um eine Durchbrechung geschlossenen Wandabschnitten gebildet werden, die sich in zumindest zwei Ebenen erstrecken, die z.B. in einem Winkel von 45° bis 90° zueinander liegen.

Alternativ kann das Mischelement massiv mit einer in sich geschlossenen Oberfläche z.B. einer Kugel, Pyramide, eines Zylinders, eines dreidimensionalen Ovals, eines Quaders, Würfels, Kegels, Kegelstumpfs oder Vielflächners aufweisen. Bevorzugt weist ein massives Mischelement Vorsprünge auf. Vorsprünge können z.B. in einer gewölbten Ebene enden, die ein Abrollen des Mischelements entlang Innenflächen eines Vorratsbehälters erlaubt. Z.B. können die Enden der Vorsprünge Abschnitte einer gewölbten Ebene bilden, bzw. Auflagepunkte bilden, auf denen das Mischelement entlang der Innenfläche eines Vorratsbehälters abrollt.

Es hat sich gezeigt, dass ein Mischelement den Anteil der feinen Partikel einer Größe von maximal 5 µm erhöht, die mittels eines Trägergases aus der pharmazeutischen Formulierung in das Trägergas übergehen. Das Mischelement führt bei der Fluidisierung der pharmazeutischen Formulierung in dem Trägergas zu einer Erhöhung des Gehalts an feinen Partikeln einer Größe von maximal 5 µm und daher beim Inhalieren zum erhöhten Eintrag solcher Partikel in die Lunge. Bevorzugt führt das Mischelement zur Fluidisierung von feinen Partikeln, z.B. einer Größe von maximal 4 µm, maximal 3 µm oder maximal 2 µm, die in die peripheren Bereiche einer Lunge inhaliert werden können.

Das Mischelement wird mittels eines additiven Herstellungsverfahrens aus einer härtenden Masse hergestellt, was generell auch als 3D-Druckverfahren bekannt ist. Alternativ können Mischelemente durch gesteuerte strahlungsinduzierte Härtung, z.B. Schmelzerstarrung oder Polymerisation, aus einer Vorläufermasse hergestellt werden, was generell auch als Photopolymerisation bzw. Laserlithographie bekannt ist.

Mischelemente können z.B. aus pharmazeutisch akzeptablem Kunststoff bestehen, der biologisch beständig ist oder der biologisch abbaubar ist.

Biologisch abbaubarer Kunststoff ist z.B. Polylactid, Polyglycolid, Polylactid-co-glycolid (PLGA). Ein biologisch beständiger Kunststoff ist z.B. EVA oder PMMA.

Bevorzugt weisen die Trägerpartikel der Formulierung eine einheitliche Größe von maximal 500 µm, z.B. 50 bis 500 µm oder bis 400 µm oder bis 350 µm in der längsten Erstreckung, und eine einheitliche Form auf. Dabei ist eine einheitliche Form generell jeweils eine identische dreidimensionale Gestalt. Es hat sich gezeigt, dass eine einheitliche Größe und Form der Trägerpartikel die Fluidisierung der Trägerpartikel und des Wirkstoffs, der an den Trägerpartikeln anhaften kann, durch einen Gasstrom und/oder die Freisetzung von partikelförmigem Wirkstoff von den Trägerpartikeln innerhalb der Atemwege verstärkt.

Die Trägerpartikel werden bevorzugt ebenfalls mittels eines additiven Herstellungsverfahrens hergestellt, z.B. durch ein 3D-Druckverfahren oder durch Photopolymerisation.

Die Trägerpartikel bestehen bevorzugt aus Material, das von einem menschlichen Körper abgebaut werden kann, z.B. nach Einbringen von Trägerpartikeln in die oberen Atemwege oder in die Lunge. Beispiele für Material, aus dem Trägerpartikel bestehen können, sind Polylactid, Polyglycolid, Polylactid-co-glycolid (PLGA), Zucker, insbesondere Glukose und Laktose, Zuckeralkohole, insbesondere Mannitol, Cellulose, Cellulosederivate, z.B. Hydroxypropylcellulose, Hydroxyethylcellulose, Hydroxypropylmethylcellulose, Gelatine, Alginat, Agarose, Carrageen, und Mischungen aus zumindest zweien dieser.

Die Trägerpartikel weisen bevorzugt einheitlich dieselbe Form und dieselbe Größe auf. Die Form kann eine sein, wie sie mit Bezug auf das Mischelement beschrieben ist, jedoch mit der Größe von maximal 500 µm, z.B. 50 bis 300 µm, in jeder Dimension. Bevorzugt werden die Trägerpartikel, die dieselbe Größe und Form bzw. die die identische Gestalt aufweisen, durch ein additives Herstellungsverfahren hergestellt, insbesondere mittels eine photolithographischen Verfahrens oder mittels eines 3D-Druckverfahrens.

Die Erfindung stellt auch einen Inhalator bereit, in dessen Strömungsweg, z.B. in einer Dispergierkammer darin, ein Mischelement enthalten ist. Dabei wird eine Formulierung aus Trägerpartikeln und Wirkstoff in dem Abschnitt des Strömungswegs, in dem das Mischelement enthalten ist, mit dem Mischelement in Kontakt gebracht. Dieser Abschnitt des Strömungswegs kann z.B. dessen Dispergierkammer sein. Das Mischelement ist generell in dem Abschnitt des Strömungswegs frei beweglich und wird z.B. durch eine Auslassöffnung mit einem Querschnitt zurückgehalten, der kleiner als das Mischelement ist.

Generell kann der Wirkstoff eine Kombination von zumindest zwei Wirkstoffen sein.

Bevorzugt wird die aerodynamische Größe von Partikeln wurde mit Apparat E gemäß European Pharmacopeia 9.0 bestimmt.

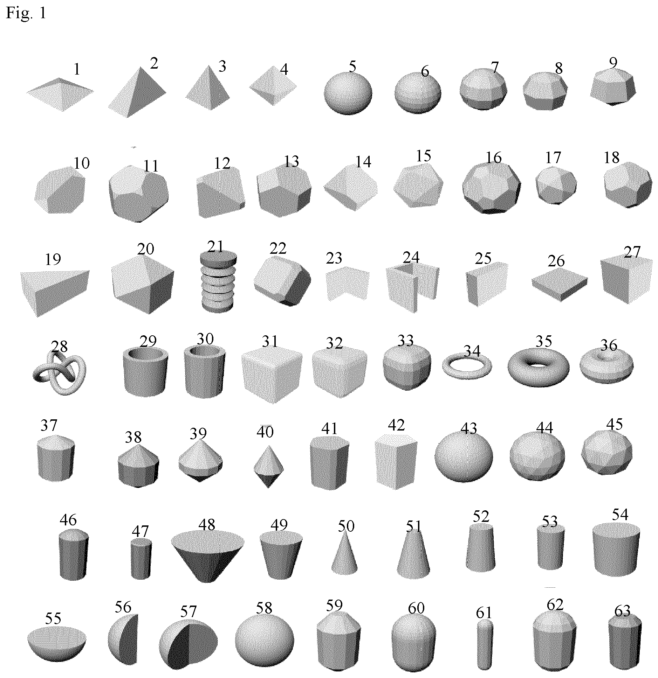

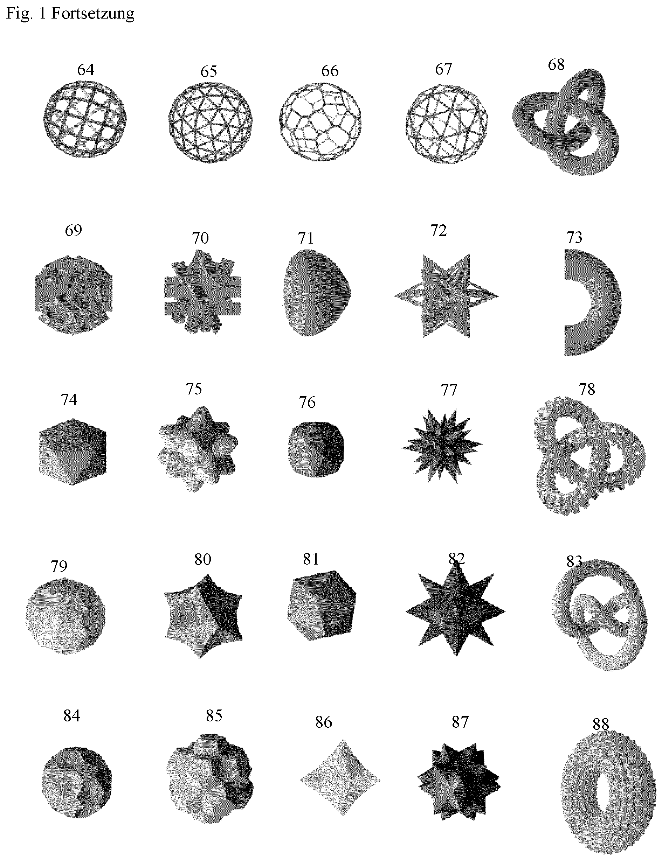

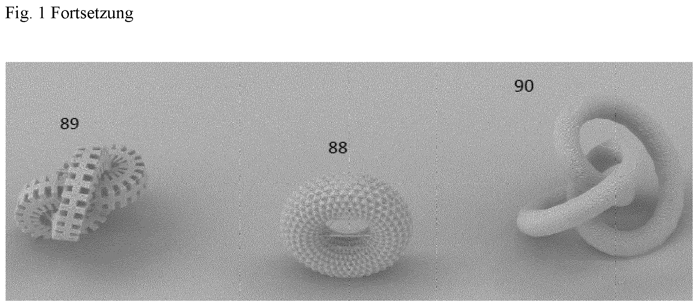

Die Erfindung wird nun genauer anhand eines Beispiels und mit Bezug auf die Figur 1 beschrieben, die beispielhafte Formen für Mischelemente und für Trägerpartikel zeigt.

Die Fig. 1 zeigt Formen für das Mischelement und für Trägerpartikel. Formen, die massiv sind bzw. keinen Hohlraum aufweisen, sind z.B. die Pyramiden 1 bis 4, die kugelförmigen Vielflächner 5 bis 20 sowie 43 bis 45 und 74 sowie 76, 58 sowie 79 oder 81, der Zylinder 21 sowie Zylinder 46 und 47 und 53 bis 54 mit gefasten Stirnflächen, die Würfel 22, 27 und 31 bis 33, der L-förmige Winkel 23, der U-förmige Winkel 24, deren Kantenlängen bevorzugt gleich lang sind, die Platten 25 und 26, die Zylinder mit zulaufenden Stirnflächen 37 bis 40 und 59 bis 63, die Quader 41 und 42 mit fünfeckiger oder mehreckigem Querschnitt und ebenen, parallelen Stirnflächen, Kegel und Kegelstümpfe 48 bis 52, massive Halbkugel 55, massive Viertelkugel 56, massive 2/3-Kugel 57, eine Kugelform mit Vorsprüngen 69 oder 70 oder Kugelform 72 mit Vorsprüngen, die Durchbrüche aufweisen, oder Kugelform 75 oder 85 mit stumpfen Vorsprüngen, ein halber Ring 73 mit z.B. rundem Querschnitt, Kugelform 77 oder 82 oder 86 und 87 mit nach außen stehenden Stacheln, kugelförmige Vielflächner 80 und 81, und kugelförmige Vielflächner 84 mit vorstehenden Kanten zwischen konkaven Oberflächenabschnitten.

Formen, deren Wände einen Hohlraum umfassen, sind z.B. Hohlzylinder 29 und 30, Ringe 34 bis 36 und 88, optional mit zackenförmigen Vorsprüngen, Gitterkugeln 64 bis 67, die aus kugelschalenförmig angeordneten Wänden bestehen, die zwischen sich Durchbrüche aufspannen,
und bevorzugt die Formen 28, 68, 78, 83, 89 und 90, die aus zumindest einem in sich verschlungenen Streifen bestehen, der sich in zumindest zwei in einem Winkel von 60 bis 90° aufeinander stehenden Ebenen erstreckt und dessen nach außen gewandte Flächen konvex gebogen sind und eine zumindest abschnittsweise, bevorzugt eine kontinuierliche kugelförmige Abrollfläche bilden, wobei der zumindest eine Streifen einen Hohlraum umfasst und Durchbrüche zwischen Abschnitten des Streifens aufspannt. Der in sich verschlungene Streifen kann einen runden oder eckigen Querschnitt aufweisen. Die Form 90 ist auch als rollender Knoten bekannt.

### Beispiel: Fluidisierung von Salbutamolsulfat auf Laktose

Als Beispiel für einen Wirkstoff wurde Salbutamolsulfat, mikronisiert (SBS, Lusochimica S.p.A, Italien), in Mischung mit Laktose zur Inhalation (InhaLac 120, Meggle, Wasserburg, Deutschland) verwendet. Das Mischelement wurde aus fadenförmigem Polylactid oder Polyvinylalkohol (beide von Ultimaker B.V, Utrecht, Niederlande) durch Aufschmelzen und Dosieren der Schmelze nach vorbestimmtem Muster mittels 3D-Drucks oder durch ein photolithographisches Verfahren aus einer durch Licht polymerisierbaren Vorläufermasse der Polymere mit den Formen hergestellt, die als Nr. 88, Nr. 89 bzw. Nr. 90 in Fig. 1 gezeigt sind. Die Größe der Mischelemente entlang ihrer jeweils maximalen Erstreckung betrug 7 mm. Das Mischelement der Form 88 weist einen zentralen Hohlraum auf, der an zwei einander gegenüberliegenden Durchbrechungen offen ist. Das Mischelement der Form Nr. 90 weist zwischen bogenförmigen Wänden Hohlräume auf, die an mehreren Durchbrechungen, die von den Wänden eingefasst sind, offen sind. Die Mischelemente der Form Nr. 88 und Nr. 90 weisen Wände auf, deren dem Hohlraum gegenüberliegende Flächen konvex gebogen sind und ein Abrollen entlang einer Innenfläche z.B. einer Mischkammer fördern.

Das Mischelement der Form Nr. 89 weist von den Wänden eingefasst Hohlräume auf und Wände, deren den Hohlräumen gegenüberliegende Flächen konvex gebogen sind und ein Abrollen entlang einer Innenfläche z.B. einer Mischkammer fördern. Die konvex gebogenen Flächen der Mischelemente der Formen Nr. 90 und Nr. 89 sind dabei kontinuierlich.

Für die pharmazeutische Formulierung wurden bei 20-25°C, 30-65 % rel. Luftfeuchte die Laktose (InhLac 120) und der Wirkstoff durch ein Sieb mit 355 µm Maschenweite gegeben und anschließendes bei einer Geschwindigkeit von 500 Upm (Picomix, Hosokawa Alpine, Augsburg), zweimal je 60 s mit einmaligem Sieben (355 µm Maschenweite) dazwischen, gemischt.

Diese Mischung aus dem Wirkstoff und der Laktose wurde entsprechend einer Ausführungsform fluidisiert, wobei das Mischelement in die Dispergierkammer und damit in den Strömungsweg des Inhalators eingebracht war.

Von der Formulierung wurden je 20,0 mg ohne zugesetztes Mischelement in Kapseln (Vcaps Plus, Größe 3, Lonza, Basel) gefüllt. Die Kapseln wurden einzeln in einen Pulverinhalator, erhältlich unter der Bezeichnung "Twister" von Aptar, Louveciennes, Frankreich, zum Messen des nach Fluidisierung erzeugten Feinanteils des Wirkstoffes eingebracht. Der Pulverinhalator wurde an einen Impaktor angesetzt, durch welchen ein mithilfe einer Vakuumpumpe erzeugter Luftstrom gezogen wurde, dessen Flussrate mittels eines digitalen Durchflussmessgeräts (Modell DFM3, Copley Scientific, Nottingham, England) auf die einem Druckabfall von 4 kPa über den Inhalator korrespondierenden Flussrate, bestimmt am Dosissammelrohr entsprechend Ph. Eur 9.0, eingestellt wurde. Ein gesteuertes Ventil wurde auf eine Öffnungszeit eingestellt, die bei der Strömungsgeschwindigkeit ein Luftvolumen von 4 L ergab, entsprechend Ph. Eur.

Die aerodynamische Größenverteilung von Partikeln wurde mittels eines Next-Generation-Pharmaceutical-Impactor (Apparat E gemäß European Pharmacopeia 9.0) bestimmt. Die abgeschiedene Wirkstoffmenge in den einzelnen Abschnitten des Apparats E wurde nach der Analyse mit Wasser gelöst und separat per HPLC (RP18-Säule, Detektion bei 220 nm, mobile Phase: 22% Acetonitril, 78% Puffer aus 2,87 g/L Natriumheptasulfonat, 2,50 g/L Kaliumhydrogenphosphat, pH 3,65, eingestellt mit 85% Orthophosphorsäure, 25°C, Flussrate 0,89 mL/min, 10 µL Probenvol.) auf den Gehalt an Wirkstoff analysiert. Mithilfe der Copley Inhaler Testing Data Analysis Software 3.0 (Copley Scientific Ltd.) wurden die Feinpartikelmasse und Feinpartikelfraktion (bezogen auf die abgegebene Dosis) aus der aerodynamischen Partikelgrößenverteilung (entsprechend PhEur) berechnet.

Es hat sich gezeigt, dass im Vergleich zur Formulierung ohne Mischelement eine erfindungsgemäße Formulierung, die mit einem Mischelement in der Dispergierkammer des Inhalators verwirbelt wurde, zu einem höheren Anteil von Wirkstoffpartikeln mit aerodynamischen Größen von 5 µm und einer Verschiebung der aerodynamischen Partikelgrößenverteilung hin zu einem höheren Anteil von Wirkstoffpartikeln < 3µm sowie < 2µm führt (Feinanteil der Dosis/Feinpartikelfraktion).

Die Tabelle zeigt die gemessenen Massen und Änderungen in % in Bezug auf die Formulierung ohne Mischelement.

| Form des Mischelements | Feinpartikeldosis [µg] | Feinpartikel < 5 µm [%] | Feinpartikel < 3 µm [%] | Feinpartikel < 2 µm [%] | MMAD [µm] |
|---|---|---|---|---|---|
| Ohne Mischelement | 59 ± 6 | 21,7 ± 2,7 | 19 ± 2,6 | 13 ± 2,2 | 1,847 |
| Form Nr. 90 | 71 ± 5 | 24 ± 1 | 21 ± 0,7 | 15 ± 0,25 | 1,759 |
| Würfel klein | 68 ± 5 | 23 ± 1,2 | 21 ± 1,1 | 15 ± 0,8 | 1,720 |
| Würfel mittel | 66 ± 1,5 | 22 ± 0,2 | 20 ± 0,1 | 14 ± 0,1 | 1,752 |
| Würfel groß | 66 ± 5 | 23 ± 1,3 | 20 ± 1,4 | 15 ± 1 | 1,722 |

| | | | | | |
|---|---|---|---|---|---|
| MMAD = massenmittlerer aerodynamischer Durchmesser | | | | | |

Der kleine Würfel hat eine Kantenlänge von 3,8 mm, der mittlere Würfel eine Kantenlänge von 4,8 mm und der große Würfel eine Kantenlänge von 5,8 mm.

Alternativ wurde der pulverförmigen pharmazeutischen Formulierung aus Laktose und Wirkstoff je ein Mischelement pro Kapsel zugesetzt und diese Kapsel wurde in die Dispergierkammer des Inhalators eingebracht und dort anschließend im Gasstrom fluidisiert.

## Patentansprüche

1. Pulverförmige pharmazeutische Formulierung zur Inhalation, die einen partikelförmigen Wirkstoff in Mischung mit Trägerpartikeln aufweist, mit zumindest einem Mischelement, das eine Größe von zumindest 1 mm in einer ersten Dimension und in einer zweiten und dritten Dimension jeweils eine Größe von zumindest 50% der Größe der ersten Dimension aufweist, **dadurch gekennzeichnet, dass** das Mischelement mittels eines additiven Herstellungsverfahrens aus einer härtenden Masse oder durch gesteuerte strahlungsinduzierte Härtung aus einer Vorläufermasse hergestellt ist.

2. Pulverförmige pharmazeutische Formulierung nach Anspruch 1, **dadurch gekennzeichnet, dass** das Mischelement aus Polylactid, Polyglycolid, Polylactid-co-glycolid (PLGA), EVA oder PMMA besteht.

3. Pulverförmige pharmazeutische Formulierung nach einem der voranstehenden Ansprüche, **dadurch gekennzeichnet, dass** das Mischelement im Strömungsweg eines Inhalators angeordnet ist.

4. Pulverförmige pharmazeutische Formulierung nach einem der voranstehenden Ansprüche, **dadurch gekennzeichnet, dass** das Mischelement um einen Hohlraum angeordnete Wände aufweist, die zumindest eine zum Hohlraum offene Durchbrechung aufweisen.

5. Pulverförmige pharmazeutische Formulierung nach einem der voranstehenden Ansprüche, **dadurch gekennzeichnet, dass** das Mischelement um einen Hohlraum angeordnete Wände aufweist, die zumindest zwei an gegenüberliegenden Wänden angeordnete und zum Hohlraum offene Durchbrechungen aufweisen.

6. Pulverförmige pharmazeutische Formulierung nach einem der voranstehenden Ansprüche, **dadurch gekennzeichnet, dass** die Wände des Mischelements zwischen ihrer Außenfläche und dem Hohlraum eine Dicke von maximal 20% der Größe des Mischelements in dieser Dimension aufweisen.

7. Pulverförmige pharmazeutische Formulierung nach einem der voranstehenden Ansprüche, **dadurch gekennzeichnet, dass** das Mischelement Wände aufweist, deren dem Hohlraum gegenüberliegende Flächen konvex gebogen sind.

8. Pulverförmige pharmazeutische Formulierung nach einem der voranstehenden Ansprüche, **dadurch gekennzeichnet, dass** das Mischelement Wände aufweist, die von einem oder von zumindest zwei miteinander verbundenen, jeweils in sich um eine Durchbrechung geschlossenen Wandabschnitten gebildet werden, die sich in zumindest zwei Ebenen erstrecken, die in einem Winkel von 45° bis 90° zueinander liegen.

9. Pulverförmige pharmazeutische Formulierung nach einem der voranstehenden Ansprüche, **dadurch gekennzeichnet, dass** das Mischelement Wände einer Form aufweist, die einen Hohlraum umfasst und ein Hohlzylinder, Ring, Ring mit zackenförmigen Vorsprüngen, Gitterkugel, die aus kugelschalenförmig angeordneten Wänden besteht, die zwischen sich Durchbrüche aufspannen, ist, oder eine Form, die aus zumindest einem in sich verschlungenen Streifen besteht, der sich in zumindest zwei in einem Winkel von 60 bis 90° aufeinander stehenden Ebenen erstreckt und dessen nach außen gewandte Flächen konvex gebogen sind und eine zumindest abschnittsweise, bevorzugt eine kontinuierliche kugelförmige Abrollfläche bilden, wobei der zumindest eine Streifen einen Hohlraum umfasst und Durchbrüche zwischen Abschnitten des Streifens aufspannt.

10. Pulverförmige pharmazeutische Formulierung nach Anspruch 1, **dadurch gekennzeichnet, dass** das Mischelement massiv mit einer in sich geschlossenen Oberfläche ist und sich über die geschlossene Oberfläche erstreckende Vorsprünge aufweist.

11. Pulverförmige pharmazeutische Formulierung nach einem der voranstehenden Ansprüche, **dadurch gekennzeichnet, dass** sie in einem Inhalator enthalten ist, der einen Strömungsweg mit einem Einlass für ein Trägergas, das von dem Wirkstoff frei ist, und mit einem Auslass für Trägergas aufweist, wobei im Strömungsweg zumindest ein Mischelement beweglich enthalten ist und der Inhalator eingerichtet ist, das Mischelement zurückzuhalten und aus der Formulierung nur den Wirkstoff und Trägerpartikel auszutragen.

12. Pulverförmige pharmazeutische Formulierung nach einem der voranstehenden Ansprüche, **dadurch gekennzeichnet, dass** der partikelförmige Wirkstoff an den Trägerpartikeln nur oberflächlich anhaftet und dass die Trägerpartikel untereinander dieselbe dreidimensionale Form und dieselbe Größe und in jeder Dimension unabhängig voneinander eine Größe von 50 bis 500 µm aufweisen.

13. Verfahren zur Erzeugung von in einem Gasstrom fluidisiertem partikelförmigem Wirkstoff aus einer pulverförmigen pharmazeutischen Formulierung nach einem der voranstehenden Ansprüche durch Fluidisieren von Wirkstoff und Trägerpartikeln in Kontakt mit zumindest einem Mischelement, **dadurch gekennzeichnet, dass** das Mischelement mittels eines additiven Herstellungsverfahrens aus einer härtenden Masse hergestellt ist und dass die Trägerpartikel dieselbe Form und Größe aufweisen, die in jeder Dimension maximal 500 µm beträgt, und sie mittels eines additiven Herstellungsverfahrens aus einer härtenden Masse hergestellt sind.

14. Inhalator, der eine pulverförmige pharmazeutische Formulierung nach einem der Ansprüche 1 bis 12 enthält.

## Claims

1. Powdery pharmaceutical formulation for inhalation, which comprises a particulate active ingredient in admixture with carrier particles, with at least one mixing element which has a size of at least 1 mm in a first dimension and in a second and third dimension in each case a size of at least 50% of the size of the first dimension, **characterised in that** the mixing element is produced by means of an additive manufacturing process from a hardening mass or by controlled radiation-induced hardening from a precursor mass.

2. Powdery pharmaceutical formulation according to claim 1, **characterised in that** the mixing element consists of polylactide, polyglycolide, polylactide-co-glycolide (PLGA), EVA or PMMA.

3. Powdery pharmaceutical formulation according to one of the preceding claims, **characterised in that** the mixing element is arranged in the flow path of an inhaler.

4. Powdery pharmaceutical formulation according to one of the preceding claims, **characterised in that** the mixing element has walls arranged around a cavity which have at least one perforation open to the cavity.

5. Powdery pharmaceutical formulation according to one of the preceding claims, **characterised in that** the mixing element has walls arranged around a cavity, which walls have at least two openings arranged on opposite walls and open towards the cavity.

6. Powdery pharmaceutical formulation according to any one of the preceding claims, **characterised in that** the walls of the mixing element between its outer surface and the cavity have a thickness of at most 20% of the size of the mixing element in this dimension.

7. Powdery pharmaceutical formulation according to one of the preceding claims, **characterised in that** the mixing element has walls whose surfaces opposite the cavity are convexly curved.

8. Powdery pharmaceutical formulation according to one of the preceding claims, **characterised in that** the mixing element has walls which are formed by one or at least two interconnected wall sections which are each closed in themselves around an opening and which extend in at least two planes which lie at an angle of 45° to 90° to one another.

9. Powdery pharmaceutical formulation according to one of the preceding claims, **characterised in that** the mixing element has walls of a shape which comprises a cavity and is a hollow cylinder, ring, ring with serrated projections, lattice sphere which consists of walls arranged in the shape of spherical shells which span apertures between them, or a shape which consists of at least one intertwined strip which extends in at least two planes which are at an angle of 60 to 90° to one another and the outwardly facing surfaces of which are convexly curved and which has an at least sectional shape which is at least partially curved, which consists of at least one intertwined strip which extends in at least two planes at an angle of 60 to 90° to one another and whose outwardly facing surfaces are convexly curved and form a spherical rolling surface at least in sections, preferably a continuous spherical rolling surface, wherein the at least one strip comprises a cavity and spans openings between sections of the strip.

10. Powdery pharmaceutical formulation according to claim 1, **characterised in that** the mixing element is solid with a closed surface and has projections extending over the closed surface.

11. Powdery pharmaceutical formulation according to any one of the preceding claims, **characterised in that** it is contained in an inhaler having a flow path with an inlet for a carrier gas free of the active ingredient and with an outlet for carrier gas, wherein at least one mixing element is movably contained in the flow path and the inhaler is adapted to retain the mixing element and to discharge only the active ingredient and carrier particles from the formulation.

12. Powdery pharmaceutical formulation according to one of the preceding claims, **characterised in that** the particulate active ingredient adheres only superficially to the carrier particles and **in that** the carrier particles have the same three-dimensional shape and the same size as one another and a size of 50 to 500 µm in each dimension independently of one another.

13. Process for the production of particulate active ingredient fluidised in a gas stream from a powdery pharmaceutical formulation according to one of the preceding claims by fluidising active ingredient and carrier particles in contact with at least one mixing element, **characterised in that** the mixing element is produced from a hardening mass by means of an additive manufacturing process and **in that** the carrier particles have the same shape and size, which is at most 500 µm in each dimension, and they are produced from a hardening mass by means of an additive manufacturing process.

14. An inhaler comprising a powdered pharmaceutical formulation according to any one of claims 1 to 12.

## Revendications

1. Formulation pharmaceutique en poudre pour inhalation, comprenant une substance active particulaire en mélange avec des particules de support, avec au moins un élément de mélange qui a une taille d'au moins 1 mm dans une première dimension et, dans une deuxième et une troisième dimension, respectivement une taille d'au moins 50 % de la taille de la première dimension, **caractérisée en ce que** l'élément de mélange est fabriqué au moyen d'un procédé de fabrication additif à partir d'une masse durcissante ou par durcissement induit par rayonnement contrôlé à partir d'une masse précurseur.

2. Formulation pharmaceutique en poudre selon la revendication 1, **caractérisée en ce que** l'élément de mélange est constitué de polylactide, de polyglycolide, de polylactide-co-glycolide (PLGA), d'EVA ou de PMMA.

3. Formulation pharmaceutique en poudre selon l'une quelconque des revendications précédentes, **caractérisée en ce que** l'élément de mélange est disposé dans le trajet d'écoulement d'un inhalateur.

4. Formulation pharmaceutique en poudre selon l'une des revendications précédentes, **caractérisée en ce que** l'élément de mélange présente des parois disposées autour d'une cavité, qui présentent au moins un ajour ouvert vers la cavité.

5. Formulation pharmaceutique en poudre selon l'une quelconque des revendications précédentes, **caractérisée en ce que** l'élément de mélange comprend des parois disposées autour d'une cavité, lesdites parois comprenant au moins deux ajours disposés sur des parois opposées et ouverts sur la cavité.

6. Formulation pharmaceutique en poudre selon l'une quelconque des revendications précédentes, **caractérisée en ce que** les parois de l'élément de mélange présentent, entre leur surface extérieure et la cavité, une épaisseur inférieure ou égale à 20% de la taille de l'élément de mélange dans cette dimension.

7. Formulation pharmaceutique en poudre selon l'une quelconque des revendications précédentes, **caractérisée en ce que** l'élément de mélange présente des parois dont les surfaces opposées à la cavité sont courbées de manière convexe.

8. Formulation pharmaceutique en poudre selon l'une quelconque des revendications précédentes, **caractérisée en ce que** l'élément de mélange présente des parois formées par une ou par au moins deux parties de paroi reliées entre elles, chacune fermée sur elle-même autour d'une ouverture, qui s'étendent dans au moins deux plans qui forment un angle de 45° à 90° l'un par rapport à l'autre.

9. Formulation pharmaceutique en poudre selon l'une des revendications précédentes, **caractérisée en ce que** l'élément de mélange présente des parois d'une forme qui comprend une cavité et qui est un cylindre creux, un anneau, un anneau avec des saillies en forme de dents, une sphère en treillis qui se compose de parois disposées en forme de cuvette sphérique qui définissent entre elles des ouvertures, ou une forme, qui consiste en au moins une bande entrelacée qui s'étend dans au moins deux plans placés l'un sur l'autre à un angle de 60 à 90° et dont les surfaces tournées vers l'extérieur sont courbées de manière convexe et forment une surface de roulement sphérique au moins par sections, de préférence continue, ladite au moins une bande comprenant un espace creux et définissant des ouvertures entre des sections de la bande.

10. Formulation pharmaceutique en poudre selon la revendication 1, **caractérisée en ce que** l'élément de mélange est plein avec une surface fermée sur elle-même et présente des saillies s'étendant sur la surface fermée.

11. Formulation pharmaceutique en poudre selon l'une quelconque des revendications précédentes, **caractérisée en ce qu'**elle est contenue dans un inhalateur qui présente un trajet d'écoulement avec une entrée pour un gaz porteur qui est exempt du principe actif et avec une sortie pour le gaz porteur, au moins un élément de mélange étant contenu de manière mobile dans le trajet d'écoulement et l'inhalateur étant adapté pour retenir l'élément de mélange et pour évacuer de la formulation uniquement le principe actif et des particules porteuses.

12. Formulation pharmaceutique en poudre selon l'une des revendications précédentes, **caractérisée en ce que** la substance active particulaire n'adhère que superficiellement aux particules de support et **en ce que** les particules de support présentent entre elles la même forme tridimensionnelle et la même taille et, dans chaque dimension, indépendamment les unes des autres, une taille de 50 à 500 µm.

13. Procédé de production d'une substance active particulaire fluidisée dans un courant gazeux à partir d'une formulation pharmaceutique en poudre selon l'une des revendications précédentes, par fluidisation de la substance active et de particules de support en contact avec au moins un élément de mélange, **caractérisé en ce que** l'élément de mélange est fabriqué à partir d'une masse durcissante au moyen d'un procédé de fabrication additif et **en ce que** les particules de support ont la même forme et la même taille, qui est au maximum de 500 µm dans chaque dimension, et elles sont fabriquées à partir d'une masse durcissante au moyen d'un procédé de fabrication additif.

14. Inhalateur contenant une formulation pharmaceutique en poudre selon l'une quelconque des revendications 1 à 12.
